# EUROPEAN PATENT APPLICATION

(11) **EP 3 501 576 A1**
(43) Date of publication of application: **26.06.2019**
(21) Application number: 17208334.7
(22) Date of filing: 19.12.2017
(51) Int. Cl.: A61M 5/32, A61M 5/46, A61M 5/142, A61M 5/158

(54) **A NEEDLE INSERTION AND RETRACTION MECHANISM FOR A MEDICATION DELIVERY DEVICE**

(71) Applicant: Tecpharma Licensing AG, 3401 Burgdorf (CH)
(72) Inventor: Scheurer, Simon, 3006 Bern (CH); Hirschel, Jürg, 3007 Bern (CH); Hostettler, Patrick, 3415 Hasle (CH)

(57) **Abstract**

A needle insertion and retraction mechanism for an injection or infusion device is presented which comprises: a needle holder for holding a needle or cannula and a needle control element which is rotatable around a first axis. The needle control element has a groove positioned at a first distance from a surface of the needle control element that is perpendicular to the first axis, and a gearing positioned at a second distance. The mechanism further has a cam-shaft which is rotatable around a second axis that is parallel to the first axis and comprises a protrusion engaging the groove of the needle control element to form a link-motion. The needle control element is initially prevented from rotation by the link-motion, thereby keeping the needle holder in abutment with an arrester and thereby keeping the needle holder in a needle retracted position against the bias of a spring force.

The needle control element has the gearing positioned at a second distance from the surface of the needle control element and the cam-shaft comprises a gear wheel for engaging the gearing of the needle control element.

## Description

### Background

Devices for delivery of medication to the patient which are adhered to the skin of the patient, so called patch devices, have been developed either for delivery of multiple adjustable doses superimposed on a basal rate (WO0240083 A2), or for multiple fixed doses including a basal rate (WO11046950 A1). Those devices have been preliminary developed for the treatment of diabetes and are used on a daily basis, e.g. the patch device remains for hours or several days on the skin of the patient. Alternatively, patch type bolus injectors were developed also for the treatment of other diseases like cardiovascular diseases, auto-immune diseases or cancer for the delivery of a single dose as described in WO10029054 A1. Such a device is adhered to the patients' skin and activated automatically or by the use of an activation button or via a remote control system and subsequently the single bolus volume will be injected. After the injection the bolus injector is removed from the skin.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Patch pumps, patch injectors or patch type bolus injectors preferably have an internal needle insertion mechanism for insertion of a needle or a soft cannula into the skin of the patient for subcutaneous delivery of the medicament. Additionally, such a mechanism can be equipped with a needle retraction feature, to retract the needle, the soft cannula and/or or steel cannula into the device before being removed from the patient's skin. Such needle insertion mechanism inserts first a steel needle together with a soft-cannula surrounding the steel needle and the needle is subsequently retracted prior to delivery of the medication through the soft cannula such as described in WO0240083 A2. Alternatively, a steel cannula is inserted in the tissue which is also used for subsequent delivery as shown in WO05002649 A1. Such internal needle inserters either automatically insert the needle from the device, for example using an insertion spring or electromotor, or the user manually applies the force for inserting the needle. Alternatively, such devices do not have an internal insertion/retraction mechanism and fluid is delivered via an external fluid path connected to a needle that is inserted using an external needle inserter.

Such medical devices preferably also have a needle retraction mechanism which retracts the needle back into the body of the medical device once the medication has been delivered to the patient. Upon removal of such patch device from the patient's skin, the retracted needle cannot be observed and there is no risk for undesired needle stitching thereby improving the usability of the device. Preferably, the insertion and retraction of the needle is combined in one mechanism as shown in EP application Nr16175895.8. The needle for the patch injector is held by a needle holder and the needle holder is guided by the housing such that the needle can be inserted by the force of a spring acting indirectly onto the needle holder via a link-motion arrangement. The insertion and retraction of the needle is controlled by a needle control element. Before the injection starts, the needle holder is kept inside the device in a needle retracted position and abuts an arrester that is present on the needle control element which prevents the insertion of the needle. The needle control element is rotated by a cam shaft having a protrusion that engages a guiding contour that is part of the needle control element to form a link-motion arrangement between the protrusion and the contour. Rotation of the cam shaft ensures that the link-motion rotates the needle control element to release the abutment of the arrester and ensures that the needle holder can move to the inserted position. Further rotation of the cam shaft ensures that the link-motion arrangement rotates and translates the needle control element such that the needle control element can retract the needle after injection of the medication. The link-motion arrangement to control the rotation comprises complex guiding contours and limits accurate control of the needle control element and effective transfer of the torque from the cam shaft to the needle control element which is a disadvantage for a reliable needle insertion and retraction mechanism.

It is an objective of the present invention to overcome the drawbacks of the prior art and provide a more reliable and arrangement for the accurate control of the needle control element controlling the insertion and retraction mechanism.

This objective is solved by the independent claim of the present invention in that a link-motion is combined with a gearing arrangement to control the movements of the needle control element. Preferably, the link motion and the gearing arrangement between the cam shaft and the needle control element are arranged separately, preferably at two levels - or within two layers of the needle control element, and the link-motion and gearing arrangement act consecutively.

### General description

The medication delivery device has a housing comprising a bottom surface and preferably is shaped such that the bottom surface can be adhered to the skin of a patient, e.g. it preferably has a planar or slightly curved or anatomically shaped bottom surface. The bottom surface comprises an adhesive layer which provides the attachment to the skin of the patient. A patient removes the device from a packaging and removes a protective layer from the adhesive layer prior to attachment to the skin. The medication delivery device preferably has an optical indication means which can indicate to a user that the device is ready for injection. The device preferably has a separate button for starting the device which can be on a top cover of the device or a switch is present on the bottom surface and skin attachment preferably activates the device. As an alternative, also the release of the protection liner for the adhesive layer can activate an electrical circuit but the start of delivery is only enabled after attachment to the skin. The control system of the device is such that the medication cannot be delivered before attachment to the skin.

The medication delivery device comprises several parts or units:
- Preferably the medication delivery device already comprises a cartridge or reservoir with the medication, e.g. is a prefilled device. As an alternative, the cartridge with medication is inserted by the user prior to use. The cartridge is shaped as a cylinder with a shoulder on one end that comprises a septum. The septum closes the cartridge on the one end and can be pierced by a needle or spike. The other end of the cartridge is open and can be filled with a liquid medicament before closing the open end with a plug or a stopper. The medicament is present between the septum and the stopper. Preferably such cartridges are made from glass and the stopper and septum are made from a suitable pharmaceutical grade rubber. Alternatively, first the plug or stopper is positioned in the open end of the cartridge which is subsequently filled from the end of the cartridge comprising the shoulder and finally the shoulder section is closed by a septum with a crimp.
- The medication delivery device comprises a drive mechanism comprising an electronic control system. The drive mechanism is powered preferably by a battery for controlling an electromotor that is also part of the drive mechanism. Furthermore the drive mechanism preferably comprises means to advance the plug or stopper present in the reservoir or cartridge. For example a piston rod is advanced by a gearing arrangement that is driven by rotation of the electromotor and the end of the piston rod abuts the plug which is thus advanced by the piston rod to expel the medicament from the cartridge.
- A housing, preferably an outer housing comprising a top cover preferably with a button and a viewing window for viewing the medicament in the cartridge. The housing furthermore comprises a bottom surface module comprising the bottom surface, preferably, a capacitive sensor, an adhesive layer and a cover or peel foil for the adhesive. After removing the cover foil of the adhesive, the device can be attached to the patient's skin. The housing comprises compartments preferably separated by walls oriented vertical to the bottom surface. Furthermore the housing preferably comprises guiding elements for guiding, for example a piston rod, a needle holder, or a needle control element.
- A fluid path unit: This unit comprises a cannula or needle, and a spike which are connected to each other via a tubing. The cannula or needle is intended for insertion into the patient's skin and the spike is intended for insertion through the septum of the reservoir such that a fluid connection is established between the cartridge and the patient via the tubing.
- A needle insertion and retraction mechanism which controls the insertion and retraction of the needle.

The needle is inserted before the drive mechanism delivers the medication to the patient. The needle retraction mechanism retracts the needle from the patient's skin after the medication has been delivered.
- A spike inserter mechanism for insertion of the spike through the septum of the cartridge and thereby establishing a fluid connection with the medicament. The spike insertion mechanism may be combined with or driven by the needle insertion mechanism but can also be designed as a separate mechanism. Alternatively, the spike inserter mechanism may drive the needle inserter mechanism.
- The different sub-units in the device provide different functionalities which are either coupled to each other electronically and/or mechanically to provide a reliable and easy functioning of the device. Therefore the medication delivery device comprises at least one coupling mechanism which preferably couples the rotation of the electromotor first to the needle insertion mechanism, followed by powering the drive mechanism and finally the electromotor is coupled to the needle retraction mechanism. The coupling mechanism preferably comprises a cam shaft.

The needle insertion and retraction mechanism for an injection or infusion device, or an injection/infusion device comprising the needle insertion and retraction mechanism according to the present invention is presented in the following.

The needle insertion and retraction mechanism comprises a needle holder for holding a needle or cannula. The needle or cannula is hollow with one sharp end that is intended for insertion into the patient. The needle holder preferably is permanently coupled to, and partially encloses the needle and has guiding means for guiding the needle holder with respect to the housing such that the needle holder performs a linear movement during insertion. The linear movement is preferably perpendicular to the bottom surface of the device. The guiding means is preferably shaped as at least one protrusion that engages at least one guide slot in the housing. Additionally, the needle holder may have second guiding means for engaging a drive part that is preferably slideable or rotatable arranged with respect to the housing. Sliding or rotating the drive part can, via the engagement with the second guiding means of the needle holder, drive the linear movement of the needle holder with respect to the housing during needle insertion. Such an engagement between the slideable or rotatable drive part and the second guiding means of the needle holder can be established as a link-motion system between the drive part and the needle holder.

The needle insertion and retraction mechanism furthermore comprises a needle control element which is rotatable around a first axis, preferably with respect to a housing or housing part of the injection or infusion device. The needle control element comprises a groove or recessed section, and the groove, or bottom of the groove, is positioned at a first distance from a surface of the needle control element that is perpendicular to the first axis. The surface of the needle control element is preferably the top surface of the needle control element and the recessed section, preferably designed as a groove, is shaped or molded into the top surface. Alternatively, the groove or recessed section is present in a first layer of the needle control element, the first layer being arranged perpendicular to the first axis.

The needle insertion and retraction mechanism further comprises a cam-shaft which is rotatable around a second axis which is preferably oriented parallel to the first axis. Preferably the first and second axis are off-set to each other. The cam shaft comprises a protrusion which is oriented parallel to the second axis and protrudes along the second axis from a surface, preferably an end surface of the cam-shaft. The protrusion of the cam shaft is preferably positioned radially displaced from the second axis. The protrusion is dimensioned such that it can engage the groove or recessed section of the needle control element to form a link-motion between the needle control element and the cam shaft. The protrusion of the cam shaft is preferably a pin with a circular cross section, alternatively elliptical or rectangular shaped pins can be used for establishing the link-motion. The cam shaft, and therewith the protrusion is positioned such in the groove or recessed section that the needle control element is prevented from rotation around the first axis. Preferably the protrusion abuts a side wall of the groove thereby preventing the rotation of the needle control element. The needle holder is thereby kept in abutment with an arrester that is part of the needle control element and thereby keeps the needle holder in a needle retracted position against the bias of a force that intends to move the needle holder from the needle retracted position to the needle inserted position. The force thus pushes the arrester of the needle control element in abutment with the needle holder. This force is provided by a resilient element, a spring element, compressed gas or a resilient element. The spring element can be a leaf spring, a compression spring, a leg spring, a spiral spring or a torsional spring. The arrester of the needle control element is preferably integrated with or molded into the needle control element as a surface that is designed to abut a counter surface of the needle holder. The surface and counter surface are preferably oriented perpendicular to, or slightly tilted, with respect to insertion direction. The needle control element furthermore has a gear rack that is present in a second recess that is positioned at a second distance from the surface, preferably the top surface of the needle control element. Alternatively, the gear rack is recessed in a second layer of the needle control element. The second layer is preferably oriented perpendicular to the first axis and parallel to the first layer. The first and second layers are connected to each other in a series arrangement and form a solid body for the needle control element. The gear rack preferably comprises gear teeth that are oriented parallel to the second axis of the needle control element and preferably start at and protrude from the top surface, and finish at the second distance from the top surface.

Furthermore, the cam-shaft comprises a gear wheel for engaging the gear rack of the needle control element. The gear wheel is preferably part of and positioned along the second axis of the cam-shaft. The gear wheel preferably has radially extending teeth oriented parallel to the second axis and the teeth are designed as complementary to the teeth of the gear rack of the needle control element.

The advantage of having two driving mechanisms for driving, preferably rotating the needle control element leads to a better and more precise control of the rotation of the needle control element. For releasing the needle insertion mechanism relatively small angles of rotation are required to release the abutment between the needle control element and the needle holder. For the needle retraction, larger angles of rotation are required for the needle control element. Both rotation modes are supported by the present invention either by the link-motion or by the gearing arrangement. In the prior art, only one control system is provided requiring a sole link motion that is complicated and less accurate or efficient in transmission of torque from the cam shaft to the needle control element. The two rotation modes can be activated consecutively, e.g. first the link-motion is operated followed by the gearing engagement or vice versa, the gearing engagement is activated first followed by the link-motion.

An injection or infusion device comprising a housing and a needle insertion and retraction mechanism, the housing can be an inner housing or an outer housing or the inner and/or outer housing comprises several parts that can be assembled to the housing of the device. The needle insertion and retraction mechanism comprises a needle holder for holding a needle or cannula and a needle control element which is rotatable with respect to the housing around a first axis. The needle control element is preferably shaped as a flat element made of a plurality of layers. In a preferred embodiment two layers that are on top of each other form the needle control element, a first layer and a second layer. Alternatively, the two layers are positioned parallel to each other or the two layers do not form a solid body but are separated from each other. The first layer comprises a groove formed in the first layer of the needle control element. The layers forming the needle control element are oriented perpendicular to the first axis. The needle insertion and retraction mechanism comprises a cam-shaft that is rotatable around a second axis with respect to the housing and the second axis is preferably parallel to the first axis. The cam shaft comprises a protrusion engaging a first section of the groove present in the first layer of the needle control element. The protrusion and the first section of the groove form a link-motion such that the needle control element is prevented from rotation around the first axis, thereby keeping the needle holder in abutment with an arrester that is part of the needle control element and thereby keeping the needle holder in a needle retracted position against the bias of a spring force. The spring force is provided by a separate spring or a resilient element that can be integrated with the housing. The needle control element furthermore has a curved gear rack formed in the second layer of the needle control element and the cam-shaft comprises a gear wheel that is engageable to -or with- the curved gear rack of the needle control element. As mentioned before, the first and second layer are next to each other and the first and second layer are in a series arrangement connected to each other.

Initially, in its pre-use state, the needle holder - and thus needle- is in the retracted position and the needle control element abuts the needle holder. Preferably, the gear wheel of the cam shaft is disengaged from the gear rack of the needle control element when the needle holder is in abutment with the arrester of the needle control element. Disengaged means that the complementary teeth of the gear rack and the gear wheel are not yet engaged. The advantage of the disengaged teeth is that - as shown below- the teeth can engage at a later stage during use and therefore the gearing engagement, controlling, for example the needle retraction cannot be activated in the pre-use state, which is advantageous as it increases the reliability of the device. The injection or infusion device comprising a housing and a needle insertion and retraction mechanism whereby the gear wheel is disengaged from the gear rack, preferably curved gear rack, when the needle holder is in abutment with the arrester of the needle control element.

The injection or infusion device comprising a housing with a needle insertion and retraction mechanism whereby the needle control element is preferably disc shaped and wherein the first layer and second layer are in a series arrangement and the cam shaft is positioned adjacent to the second layer. The flat element forming the needle control element is preferably made of at least two adjacent layers and the cam shaft is positioned next to or closest to the second layer. The first and second layers form the back and front surface of the needle control element and the cam shaft is next to the front surface of the needle control element. The first and second surfaces are preferably sandwiched together at the back surface of the second layer and the front surface of the first layer. The front surface of the needle control element is the front surface of the second layer and the back surface of the needle control element is the back surface of the first layer.

The groove or recessed section of the needle control element is preferably curve shaped and preferably partially comprises a circular section. Alternatively the groove is a linear groove. Preferably the groove has a constant depth, alternatively the bottom of the groove is sloped with respect to the plane forming the first layer. The groove in the needle control element has side walls and a bottom surface. The bottom surface of the groove is preferably distanced from the top surface of the needle control element at a first distance. The cross section of the groove is preferably rectangular shaped, U-shaped or V-shaped comprising side-walls and a bottom surface. The first section of the groove that is curved preferably has a constant width and the second section of the groove is broader compared to the first section. The injection or infusion device comprising a housing and a needle insertion and retraction mechanism wherein the first section of the groove of the needle control element is curved or partially circular shaped.

The needle insertion and retraction mechanism wherein the first distance from the surface, preferably top surface of the needle control element is above or more then the second distance from the surface of the needle control element. In other words, the groove is shaped deeper into the top surface of the needle control element then the teeth forming the gearing. Alternatively, the situation is reversed, e.g. the first distance is below the second distance. The advantage of using two engagement mechanisms that are at a different distance from the surface is that the two mechanisms are separate from each other. In other words, the first layer with the groove is positioned further away from the cam shaft compared to the second layer with the gear rack.

The needle control element is prevented from rotation by an abutment of the protrusion of the cam shaft with a side wall of the groove of the link-motion system. The needle control element releases the needle holder and injection needle during the injection phase. Before injection, after assembly of the device and during shelf life, the needle should remain in the retracted state and release of the needle by rotation of the needle control element prevented. For example during transport with temperature changes, pressure changes eventually impact movements (dropping), the needle control element needs to be rotationally secured. The link motion between the protrusion of the cam shaft and the preferably curved groove is designed such that rotational movements of, and torque on the needle control element are counteracted by the abutment between the protrusion of the cam shaft and the side wall of the groove. The cam shaft, and therewith the protrusion is temporarily restricted or limited in axial and/or rotational movements to secure the abutment. The needle insertion and retraction mechanism wherein the needle control element is prevented from rotation around the first axis by abutment of the protrusion of the cam shaft with a side wall of the first section of the groove of the needle control element.

A rotation of the cam shaft in a first direction over a first angle rotates the needle control element over a second angle due to the engagement between the link-motion of the needle control element and the protrusion of the cam shaft. During use, or after activation of the device, the cam shaft is rotated, preferably by an electromotor over the first angle such that the protrusion of the cam shaft rotates as well. During this rotation, the initial abutment between the protrusion and the side wall of the groove is released. The protrusion is preferably positioned off-axis from the second axis of the cam shaft and rotation of the cam shaft results in that the protrusion moves on a circular path curve that matches the circular path curve of the first section of the groove. The protrusion thus runs through the first section of the groove without interacting with the groove as the width, depth and curvature of the groove is adjusted to the dimensions and rotational movement of the complementary protrusion. Subsequently, the protrusion activates the link-motion engagement such that the needle control element rotates around the first axis through the second angle. Once the circular section of the groove ends on the needle control element, the circular movement of the protrusion ensures that the protrusion engages a side wall of the groove and activates the link-motion. The rotation of the needle control element preferably stops once the cam shaft has been rotated over the first angle. The needle insertion and retraction mechanism wherein a rotation of the cam shaft in a first direction through a first angle rotates the needle control element through a second angle due to the link-motion between the needle control element and the cam shaft. Preferably, the protrusion of the cam shaft rotates through or runs through the first section of the curve of the needle control element before the link motion rotates the needle control element. Preferably the first section of the groove ends where the link motion is active in rotating the needle control element.

The rotation of the needle control element through the second angle releases the abutment between the arrester of the needle control element and counter surface on the needle holder whereby the needle holder is moved from the needle retracted to a needle inserted position by the force provided by the resilient element, such as a spring. Once the abutment is released, the needle holder is free to move with respect to the housing and is guided by the housing for the linear movement from the needle retracted position to the needle inserted position. Optionally, the needle holder is releasable secured in the needle inserted position by an element on the housing or on the needle control element, for example by a resilient locking arm. The needle insertion and retraction mechanism wherein the rotation of the needle control element through the second angle releases the abutment between the arrester of the needle control element and the needle holder whereby the needle holder is moved from the needle retracted to a needle inserted position by the spring force.

A rotation of the cam shaft in the first direction through a third angle which is above the first angle brings the gear wheel of the cam shaft into a gearing engagement with the needle control element, preferably with the gear rack. Once the cam shaft is rotated further in the same rotation direction through an angle above (or beyond) the first angle, then the teeth of the gear wheel of the cam shaft start engaging the teeth of the gear rack of the needle control element. Thus the gearing arrangement which is present at the second distance (or first layer) in the needle control element is activated once the cam shaft has been rotated through an angle that is at least equal to the third rotation angle. The rotation over the first and third rotation angle is in the first rotation direction. Between rotation of the first and third rotation angles in the first rotation direction, the cam shaft may be rotated in the second rotation direction. The advantage is that by selecting the rotation angle, also the activation of the two separated engagement mechanisms can be done consecutively by the rotation of a single part, the cam shaft, with respect to the needle control element. The needle insertion and retraction mechanism wherein rotation of the cam shaft in the first direction through a third angle which is beyond the first angle brings the gear wheel of the cam shaft into a gearing engagement with the curved gear rack of the needle control element due to the link-motion.

It is preferred to release the engagement between the protrusion of the cam shaft and the link-motion, e.g. the protrusion engaging the first section of the groove, of the needle control element when the gearing engagement between the cam shaft and the needle control element has been established. The link-motion engagement could otherwise jam or block the further rotation of the needle control element by the gearing engagement. The link-motion is released, for example by broadening the groove at the first distance (or in the first layer) or providing a pocket where the protrusion of the cam shaft can move without interacting with the needle control element. Preferably, the gearing engagement is established simultaneously when the link-motion engagement is released. Optionally, the link-motion is designed such that the pin can re-engage with the groove upon further rotation of the needle control element, preferably once the gearing engagement has been released. The needle insertion and retraction mechanism whereby the link-motion between the protrusion of the cam shaft and the first section of the groove of the needle control element is released and the protrusion enters a second section of the groove. The first section and second section of the groove are connected to each other, the first section is preferably curve or circular shaped, and the second section is broader such that the second section does not interfere or engage the protrusion of the cam shaft when the gearing engagement is operative.

The gearing engagement ensures that the needle control element is rotated through an angle above the second angle such that a retraction arm preferably being part of the needle control element moves the needle holder from the needle inserted position back to the needle retracted position. After release of the link-motion, the gearing engagement controls the rotation of the needle control element, which enables a good control of the rotation of, and torque transmission to, the needle control element. This may be required if the needle holder is pushed back by the retraction arm against the spring force. The needle control element comprises an arm, a leg or a part that is designed to abut the needle holder when the needle control element is rotated through an angle that is above the second angle. Optionally, the retraction arm is part of a resilient member. Preferably, the gearing engagement enables the release of the locking mechanism used to lock the needle holder in the inserted position. The needle insertion and retraction mechanism wherein the gearing engagement rotates the needle control element beyond the second angle such that a retraction arm moves the needle holder from the needle inserted position back to the needle retracted position.

An injection device or infusion device comprising the needle insertion and retraction mechanism whereby the needle control element is engaged with a housing or a housing part of the injection or infusion device. The needle control element of the injection or infusion device is thus engaged with the housing or housing part of the injection or infusion device. The housing or housing part comprises compartments or cover parts separated by walls that are used to support parts such as the needle control element. For example, the needle control element has at least one protrusion contributing to at least one housing link-motion that preferably exists between the housing or housing part and the needle control element. Preferably, a wall that is part of the housing comprises at least one groove, key, or guide slot contributing to the housing link-motion that engages the at least one protrusion of the needle control element. The housing link-motion controls the rotation and/or translation of the needle control element. The at least one protrusion is preferably aligned with the first axis and protrudes from the top surface and/or the bottom surface. The needle insertion and retraction mechanism wherein the needle control element is engaged with the housing or a housing part of the injection or infusion device. Preferably, the needle control element has at least one protrusion oriented parallel to the first axis and preferably defining the first axis, the protrusion engaging a recess in the housing or a part of the housing to form a bearing such that the needle control element can rotate around the first axis. The recess in the housing can be circular or elongated such that the needle control element can rotate and/or axially translate.

The force that intends to move the needle holder from the needle retracted position to the needle inserted position preferably acts directly onto the needle holder, or, alternatively indirectly via a second link-motion arrangement or via a lever-arm. For example a spring such as a leaf spring or compression spring directly abuts or engages the needle holder. The opposite end of the compression spring end abuts for example a wall or cover of the housing of the injection or infusion device, or the other end of the leaf spring is connected to the housing. An example of an indirect engagement is via a lever arm abutting the needle holder, whereby the lever arm is biased by a spring member.

The needle or cannula is preferably made of steel. The cannula holder is preferably made from a plastic material and is injection molded at least partially around the needle, or the needle is inserted in the needle holder and fixed with a glue or by a press-fit engagement. Alternatively, the cannula is made from a rigid plastic material such as PEEK, POM, PC, PPSU, or a fiber reinforced plastic material. In another example, the steel needle is combined with a soft cannula surrounding the steel needle. The soft cannula and steel needle are both inserted into the skin and the soft cannula remains in the body of the patient after retraction of the steel needle.

The cam-shaft of the needle insertion and retraction mechanism is preferably designed as a cylinder having an end wall. The cylindrical shape has an opening on one side and the protrusion and gear wheel are positioned opposite to the opening along the second axis. The protrusion and gear wheel are preferably part of or shaped into the end wall of the cam-shaft. Preferably the cam-shaft extends through a passage in the housing and is rotatable with respect to the housing. The cam shaft may engage the housing directly in a press-fit or snap-fit engagement or via another element such as an O-ring. The cam shaft and wall preferably separate two compartments of the injection or infusion device and form a water tight and/or sterile barrier. On one side of the barrier, the opening in the cam shaft engages the drive mechanism of the device, on the other side of the wall, the gear wheel and protrusion engage the needle insertion and retraction mechanism. The needle insertion and retraction wherein the cam-shaft has a cylindrical shape having an opening on one side and the protrusion and gear wheel being positioned opposite to the opening along the second axis, the protrusion of the cam shaft engaging the groove of the needle control element is oriented parallel to the second axis and positioned preferably off-axis to the second axis. Preferably, the protrusion extends axially further from the end wall of the cam shaft compared to the gear wheel. The protrusion is configured to engage the groove in the first layer of the needle control element and the gear wheel is configured to engage the gear rack of the needle control element in the second layer. As an alternative, the gear wheel extends axially further from the end wall compared to the protrusion.

The needle insertion and retraction mechanism whereby the cam shaft is rotated in a second rotation direction which is opposite to the first rotation direction, the cam shaft being rotated in the second rotation direction between the first angle of rotation and third angle of rotation for the needle control element. Switching the rotation direction is part of a method for needle insertion, dispense of medicament and needle retraction. The method comprises the steps of:
Rotation of the cam shaft through the first angle in the first rotation direction to release the abutment between the arrester and the needle holder to move the needle holder from the needle retracted to the needle inserted position. When the rotation through the first angle of the cam shaft has been completed, the link motion between the cam shaft and the needle control element is active and rotates the needle control element through a second angle which releases the abutment between the needle control element and the needle holder.
Followed by the step of rotation of the power unit or electromotor in the second rotation direction to power the drive mechanism and inject the medicament present in the reservoir via the fluid path using the drive mechanism of the medication delivery device. The second rotation direction is preferably not transmitted to the cam shaft such that the link motion and/or gearing engagement is non-active. Finally the rotation direction of the cam shaft is reversed to the first rotation direction and the cam shaft is rotated through a third angle to activate the gearing engagement and de activate the link motion. As a consequence of the rotation of the cam shaft and the gearing engagement, the needle control element is rotated beyond the second angle to move the needle from the needle inserted position back to the needle retracted position using a needle retraction arm. The needle insertion and retraction mechanism wherein the cam shaft is rotated in a second rotation direction which is opposite to the first rotation direction, the cam shaft being rotated in the second rotation direction between the first angle of rotation and third angle of rotation of the cam shaft.

### Legends to the Figures:

Figure 1: Parts of the patch injector with top cover, drive mechanism and coupling mechanism
Figure 2: Parts of the patch injector with cartridge, fluid path unit and needle insertion and retraction mechanism
Figure 3a: Needle control element
Figure 3b: Needle control element, view on top surface
Figure 3c: Needle control element, side view indicating cross sectional views A and B
Figure 3d: Needle control element, cross sectional view A-A through the first layer showing groove of the motion link
Figure 3e: Needle control element, cross sectional view B-B through the second layer showing gearing
Figure 4: Camshaft
Figure 5a: Needle insertion and retraction mechanism with spike carrier, needle holder, and cam shaft and needle control element
Figure 5b: Needle insertion and retraction mechanism with spike carrier, needle holder, cam shaft and needle control element
Figure 5c: Needle insertion and retraction mechanism with spike carrier, needle holder, cam shaft and needle control element, top view
Figure 6a: Assembly of needle control element and cam shaft in the starting position, needle control element abuts the needle holder
Figure 6b: Assembly of needle control element and cam shaft starting position, cross section A-A through the first layer; protrusion of cam shaft prevents rotation of the needle control element
Figure 6c: Assembly of needle control element and cam shaft starting position, cross section B-B through the second layer; gear wheel of cam shaft is disengaged from gearing of the needle control element
Figure 7a: Assembly of needle control element and cam shaft, cam shaft rotated, cross section A-A through the first layer; protrusion of cam shaft remains in curved groove of the needle control element, needle control element is not rotated
Figure 7b: Assembly of needle control element and cam shaft, cam shaft rotated, cross section B-B through the second layer; gear wheel is disengaged from gearing
Figure 8a: Assembly of needle control element and cam shaft, cross section A-A; rotation of the cam shaft over first angle, needle control element is rotated over second angle
Figure 8b: Assembly of needle control element and cam shaft, cross section B-B; rotation of the cam shaft over first angle, gear wheel disengaged from gearing
Figure 9a: Assembly of needle control element and cam shaft, cross section A-A; rotation of the cam shaft over third angle to engage the gearing between the gear wheel of the cam shaft and the gearing of the needle control element, the motion link between the curved grooved and the protrusion is released
Figure 9b: Assembly of needle control element and cam shaft, cross section B-B; rotation of the cam shaft over third angle to engage the gearing between the gear wheel of the cam shaft and the gearing of the needle control element, the motion link between the curved grooved and the protrusion is released
Figure 10: Assembly of needle control element and cam shaft, cross section B-B, rotation of the cam such that the gearing engagement ensures that the needle control element is rotated further - the retraction arm engages the needle holder to retract the needle
Figure 11a: Needle control element in the needle retracted position, cross section A-A - first layer
Figure 11b: Needle control element in the needle retracted position, cross section B-B - second layer

### Detailed description of the invention

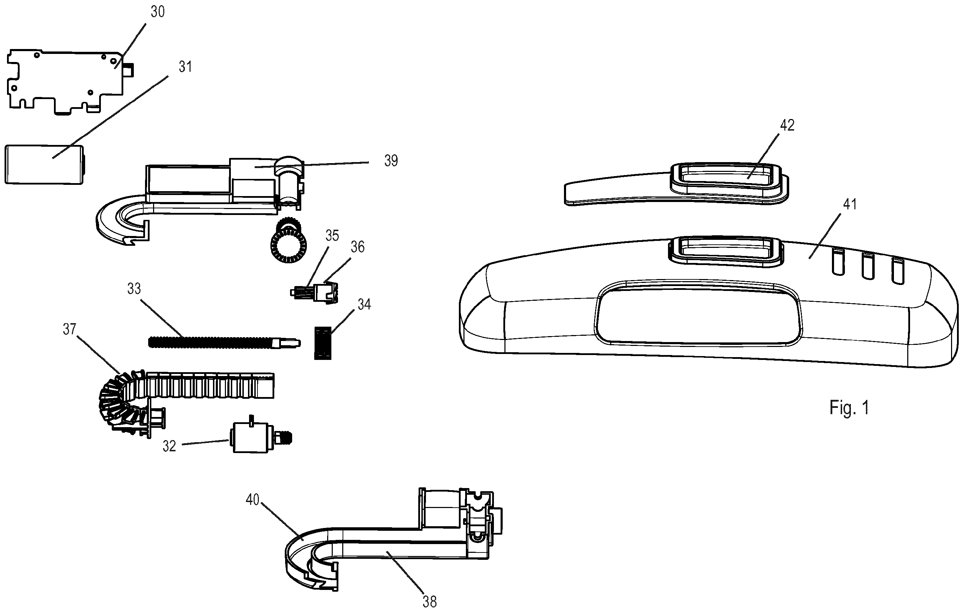

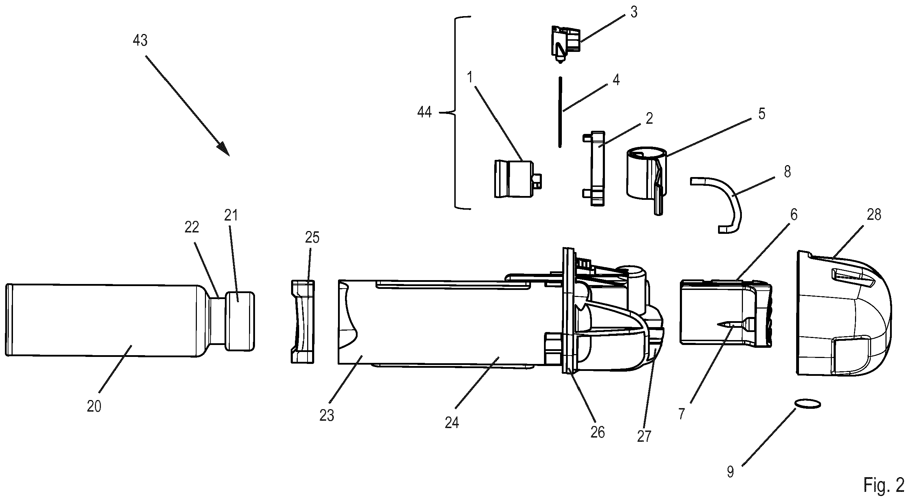

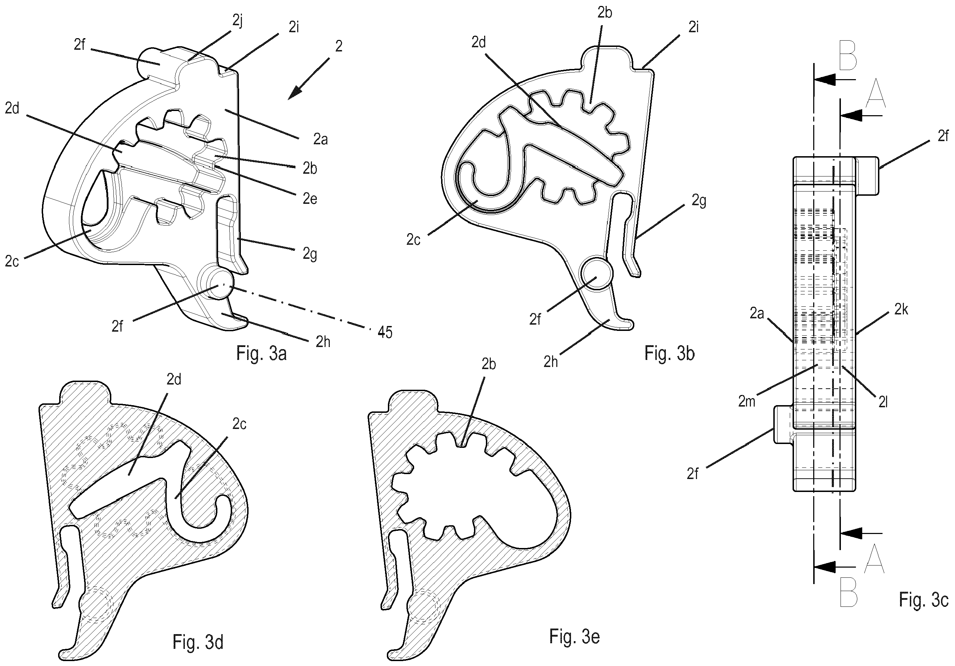

In Figures 1 and 2, an exploded view of a patch injector is presented comprising the needle insertion and retraction mechanism shown in more detail in Figures 3 to 11. The patch injector has a control unit (30) for controlling the patch injector which is powered by a battery (31). The drive mechanism of the patch injector comprises a motor (32) which may be powered by the battery (31) and which is configured to rotate a threaded rod (33) via a gear wheel (34) and shaft (35). The shaft (35) is preferably rotated via a gearing engagement with the gear wheel (34). Preferably the shaft (35) comprises a coupling member (36), and the coupling member preferably engages a cam shaft (1) such that a rotation of the motor (32) in at least one of the two rotation directions is transferred to a rotation of the cam shaft (1). The threaded rod (33) in this embodiment has an outer threading which engages an inner threading of at least a part of a piston rod (37), which is shown here as a segmented piston rod. At least one segment of the piston rod (37) has an internal threading matching the external threading of the threaded rod (33). The piston rod is prevented from rotation around its longitudinal axis by a bottom carrier part (38) and a top carrier part (39) which provide for a guidance (40) for the segmented piston rod. The segments of the piston rod (37) are connected to each other via film-links such that the piston rod (37) can bend in one direction while load can still be transferred. Rotation of the threaded rod (33) thus ensures that the piston rod (37) is advanced or retracted. The patch injector has a housing cover (41), preferably equipped with a cut out to receive a button (42) which can be used to activate the device or start the injection procedure. Optionally there are windows in the housing cover or transparent areas to display information to the patient, for example using visual indicators like LED lights informing the patient about the status of the injection procedure. The fluid path unit (43) is presented in Figure 2 and comprises a cartridge (20) having a glass barrel (optionally the barrel is made of plastic) that is open on one end and closed at the opposite end by a crimp (21) holding a not shown septum. The cartridge (20) has a shoulder (22) that is preferably used to fixate the cartridge (20) in a cartridge holder (23). Alternative fixation methods are by fixation of the crimp or fixation of the glass barrel of the cartridge to the cartridge holder (23). The cartridge holder (23) is part of a housing (24) of the fluid path unit (43), the cartridge holder (23) has an opening for receiving the shoulder section (22) with the crimp (21) of the cartridge (20). After insertion, the cartridge (20) is fixated to the housing (24) of the fluid path unit with an adequate fixation means, for example by a ring shaped cartridge fixator (25). Alternative fixation means such as glue, adhesive tapes, O-rings, resilient arms or fingers can be used as well. The cartridge (20) comprises a plug or stopper (not shown) to close the open end of the glass barrel after the cartridge has been filled with a liquid and/or solid medicament. The housing (24) of the fluid path unit comprises at least one housing wall (26) and a bottom section (27) which is used for holding and guiding the parts of the needle insertion and retraction mechanism. The fluid path unit is closed by a cover (28), creating a cavity between the housing wall (26) and the cover (28). The needle insertion and retraction mechanism is located preferably within the cavity of the fluid path unit (43).

The bottom section (27) of the housing (24) can be also form the outer housing for the patch device or there is a separate bottom cover (not shown) matching the housing cover (41) to enclose the patch injector together with the cover (28).

The needle insertion and retraction mechanism (44) comprises a cam shaft (1), a Needle Control Element (NCE) (2), a needle holder (3) which at least partially encloses a hollow needle or cannula (4) and a biasing member or spring (5), see Figure 2. The needle holder (3) is preferably made from a polymeric material that is at least partially injection molded around the needle or cannula (4). The needle holder (3) preferably has at least one protrusion or key for engaging the housing (24), the housing wall (26) or a slideable part such as a spike carrier (6). The protrusions or keys of the needle holder (3) ensure that the needle control element is guided when moving from a needle retracted to a needle inserted position, additionally the protrusions or keys may be used for transferring a driving force onto the needle holder (3) that drives the needle holder (3) from the needle retracted towards the needle inserted position which will be explained below. The needle (4) preferably is a hollow steel needle with one sharp end for penetrating the skin of the patient. The end of the needle that is opposite to the sharp end is at least partially enclosed by the needle holder (3). Alternatively, the hollow needle (4) is made from a rigid plastic material. The hollow needle (4) is connected to a spike (7) via a tubing (8). The spike (7) is preferably part of the spike carrier (6) which is slidably mounted with respect to the housing (24) or bottom section of the housing (27). The spike (7) of the spike carrier (6) is hollow with a sharp end that is aligned with the longitudinal axis of the cartridge (20) and the spike carrier (6) can be moved linearly such that the spike (7) penetrates the septum (21) that is crimped onto the cartridge. The spike and carrier are preferably produced as a single piece by injection molding of a plastic material, alternatively, the spike is made from steel and shaped as a hollow needle and glued into the carrier. Once the spike (7) has penetrated the septum, a fluid connected is established via the tubing (8) between the liquid medicament and the needle (4). The spike carrier may be equipped with a driving means for driving the needle holder (3) for example by a link-motion using the keys or protrusions of the needle holder.

The needle holder (3) can be moved from a needle retracted to a needle inserted position under the bias of the spring member (5). The spring member in this embodiment is a leaf spring but other spring configurations (compression, torsion, spiral springs) can also be used. The spring force acts between the housing, either directly onto the needle holder or indirectly via a third part. In the embodiment of Figure 2, the leaf spring (5) biases the spike carrier (6) to move the spike (7) towards the septum of the cartridge (20), e.g. the spike (7) does not puncture the septum (21) prior to activation of the device (for example during shelf life). The bias of the spring (5) acts on the needle control element (2) due to a link-motion engagement or guiding engagement between the spike carrier (6) and the needle holder (3). The biasing force of the spring (5) thus indirectly intends to move the needle holder (3) towards the needle inserted position. Alternatively, the spring force acts on a lever arm and the lever arm pushes against the needle holder (3) such that the needle holder (3) intends to move towards the needle inserted position. Prior to use, the movement of the needle holder (3) towards the inserted position is blocked, for example by the needle control element (2) as will be explained below.

The user preferably attaches the injector to the skin using an adhesive patch that is attached to the bottom of the device. After activation of the device, for example by closing the electrical circuit powered by the battery (31), the button (42) can be pushed by the user and the control unit (30) ensures, for example by rotating the electromotor (32) and cam shaft (1), that the needle holder (3) is released and the needle (4) inserted into the skin of the patient. The spring force pushes the needle through a not shown passage in the housing, bottom housing (27) or housing cover (28). The passage is preferably closed by a peel foil (9) forming a sterile barrier, e.g., during shelf life, the cavity formed by the cover (28) and housing wall (26) remains in a sterile condition. The user removes the peel foil (9) prior to insertion of the needle (4), for example by removing a not shown liner that covers the adhesive patch of the injection device before attachment to the patient's skin.

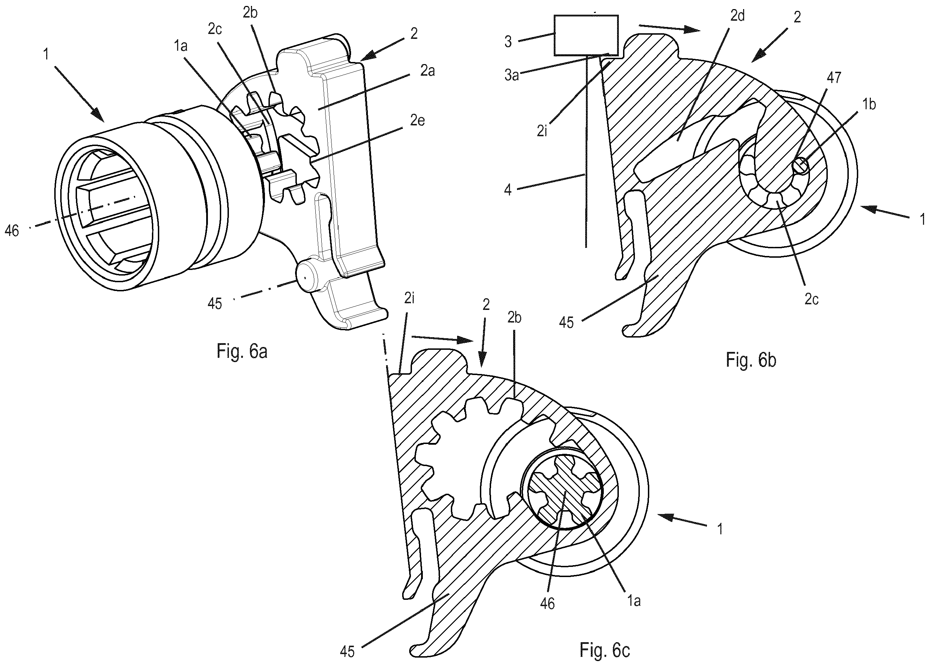

Details of the parts of insertion and retraction mechanism and the functioning of the mechanism are presented in Figures 3 to 11. In Figures 3 to 5, the needle control element, cam shaft and the assembly of the needle control element with cam shaft and spike carrier are presented respectively. In Figures 6 to 11, the functioning of the mechanism is explained. The needle control element (2), NCE is displayed in Figures 3a to 3e. The NCE (2) comprises a front surface (2a) and a back surface (2k) defining a space for a link-motion and a gearing engagement between the NCE (2) and the cam shaft (1). At least one protrusion (2f) protrudes from the front surface (2a) and the at least one protrusion (2f) engages a recess in the housing, in the cover (28) or in the housing wall (26) such that the NCE (2) can rotate and/or translate with respect to the housing wall (26). At least one protrusion (2f) protrudes from the back surface (2k) engaging a recess in the housing, cover (28) or housing wall (26) to support the rotation and/or translation of the NCE (2). Preferably, the NCE (2) rotates around a first axis (45) defined by the cylindrical protrusion (2f) that protrudes from the front surface (2a). The first axis (45) is preferably perpendicular to the front surface (2a) and preferably parallel to the longitudinal axis of the cartridge (20). The first axis is preferably perpendicular to the back surface (2k), e.g. preferably the front and back surfaces of the NCE are parallel arranged to each other.

A gear rack (2b) comprising a plurality of gear teeth (2e) is recessed from the front surface (2a). Furthermore. The NCE (2) comprises a curved groove (2c) that is U-shaped in cross section and the curved groove is recessed from the front surface (2a) such that the groove is below the gearing (2b) as viewed from the front surface (2a) of the NCE. The curved groove (2c) has a constant width and depth and preferably ends or is connected to a broadened release section (2d). The release section (2d) is preferably positioned at the same distance from the front surface (2a) of the NCE (2) as the curved grooved (2c).

The NCE (2) is a disc shaped element with a front surface (2a), a back surface (2k), see Figure 3C. The NCE in this embodiment comprises two layers, a first layer (21) and a second layer (2m). Both layers are arranged in series and are connected to each other to form the disc shaped element. The cross sectional plane A-A is within the first layer (21) whereas the cross sectional plane B-B is within the second layer (2m). The first layer (21) starts at the back surface (2k) and ends in the plane used for forming the groove (2c) whereas the second layer (2m) starts at the front surface (2a) and ends at the plane used for forming the groove (2c) The curved groove (2c) and the release section (2d) are part of or formed within the first layer whereas the gear rack (2b) is part of or formed within the second layer.

The NCE (2) has a retraction arm (2h) which extends from the needle control element and is designed to abut the needle holder (3) during retraction of the needle (4) from the skin of the patient. Furthermore the NCE (2) has an arrester surface (2i) bordered by a protrusion (2j) defining a receiving area for the needle holder (3).

Optionally, the NCE (2) comprises a locking arm (2g) which ensures that the needle holder (3) can be releasably locked in the needle inserted position. The locking arm (2g) is preferably of resilient nature and can engage a protrusion, groove or counter surface on the needle holder (3).

A view on the front surface (2a) of the NCE shows the contours of the gear rack (2b), the curved groove (2c) and the release section (2d), see Figure 3b. In Figure 3c, a side view of the NCE (2) is presented indicating the position of two cross sections, A-A and B-B, distanced from the front surface (2a) of the needle control element and the position of the first layer (21) and the second layer (2m). The cross section B-B through the second layer (2m) is closer to the front surface (Figure 3e) compared to cross section A-A through the first layer (21) as is shown in Figure 3d. This figure shows the contour of the gear rack (2b), whereas cross section A-A (Figure 3d) is further away from the front surface and presents the contours of the curved groove (2c) and the release section (2d). Depending on the distance from the front surface (2a), there is either the curved groove with the release section or the gearing available for engagement with the cam shaft (1).

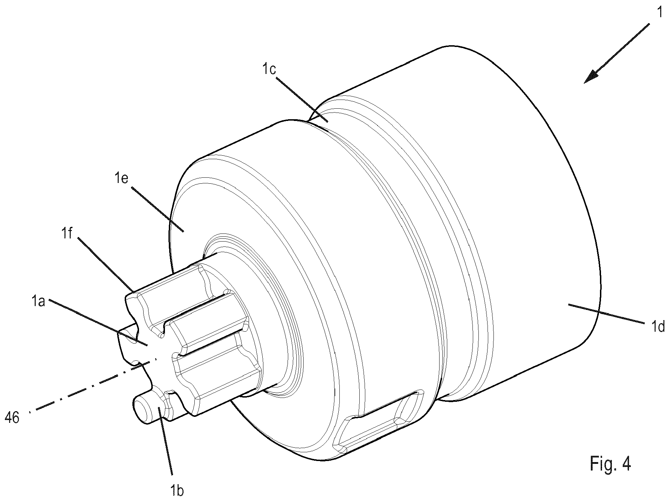

The cam shaft (1), comprises a hollow cylindrical body (1d) having a central or second axis (46), the second axis (46) preferably being parallel and off-set arranged with respect to the first axis (45). The hollow body is closed by an end wall (1e) and a gear wheel (1a) is attached to the end wall (1e), Figure 4. The gear wheel (1 a) comprises gear teeth (1f) that are complementary to the gear teeth (2e) of the gear rack (2b) and the gear teeth (1f) are preferably oriented parallel to the second axis (46) of the cylindrical body (1d). A protrusion (1b) is present on the end wall (1e) or on top of the gear wheel (a) and the protrusion is preferably oriented parallel to, and positioned off axis from the longitudinal axis (46). The protrusion (1b) preferably extends axially further from the end wall (1e) then the gear teeth (1f). The protrusion (1b) is designed to engage the curved groove (2c) of the needle control element. The cam shaft (1) is preferably engaged with the housing of the injection device or with the housing wall (26) via circumferential groove (1c). The cam shaft (1) can rotate with respect to the housing or the housing wall (26) around the second axis (46) while being axially fixated. The hollow opening (1g) of the cam shaft (1) is configured to engage a coupling member or gear wheel, for example the coupling member (36) as shown in Figures 1 and 6a. The cam shaft (1) is preferably engaged with the housing or housing wall (26) via a resilient member of the housing to establish a snap-fit engagement with the circumferential groove (1c).

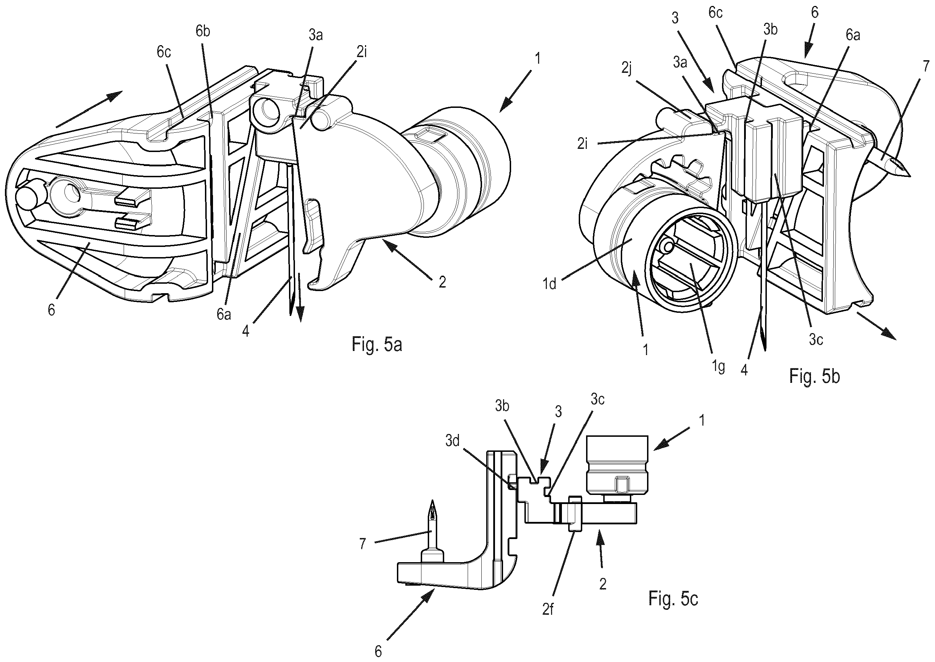

The assembly of the cam shaft (1), needle control element (2), needle holder (3) and spike carrier (6) is shown in Figures 5a to 5c. The spike carrier (6) is engaged with the housing via third guide slots (6c) such that the spike carrier can move parallel to the bottom surface of the device. The third guide slots (6c) are engaged with linear keyways on the housing (not shown). The needle holder (3) in Figure 5a is shown in the needle retracted position and a counter arrester surface (3a) abuts the arrester (2i) of the needle control element. The needle holder (3) comprises linear second guidances (3b,3c, Figure 5c) that engage linear protrusions on the housing or housing wall (not shown), such that the needle holder can move in a direction that is preferably perpendicular to the bottom section (27) of the housing or lower bottom cover of the device. The needle holder (3) furthermore has one protrusion (3d), Figure 5c), configured to engage a guide slot (6a) of the spike carrier (6), Figure 5a). The guide slot (6a) is a linear guide that is angulated with respect to the bottom surface of the device and the guide slot (6a) forms a link-motion (3d, 6a) with the protrusion (3d) of the needle holder. The spike carrier (6) is in this example biased by spring (5), see Figure 2, which intends to push the spike carrier (6) towards the cartridge (20), as indicated by the arrow in Figure 5a. A linear movement of the spike carrier (6) parallel to the bottom surface ensures that the needle holder (3) is pushed perpendicular to the bottom surface towards the needle inserted position through the link motion (3d, 6a). The needle holder (3) is thus indirectly biased by the spring (5) through the link-motion (3d, 6a). As an alternative, the spring (5) may directly act on the needle holder, for example a compression spring is position between the cover (28) and the needle holder (3), and one end of the spring directly abuts the needle holder (3).

The spike carrier (6) may have a second guide slot (6b) which is available for the protrusion (3d) of the needle holder (3) during the needle retraction procedure. The needle control element (2), the needle holder (3), the cam shaft (1) and the spike carrier (6) are all engaged with the housing or housing wall (26). The needle control element (2) is engaged with the housing via protrusions (2f) which enables that the needle control element to rotate around first axis (45); the needle holder (3) is engaged with the housing via second guidances (3b,3c) such that the needle holder can move perpendicular to the bottom of the device; the cam shaft (1) is engaged with the housing such that it can rotate in both rotation directions around the second axis (46); the spike carrier is engaged with the housing such that it can slide via third guide slot (6c) parallel to the bottom surface of the device. In Figure 5b, the assembly is shown in a position where the opening (1g) of the cylindrical body (1d) is presented that is available for engagement with a coupling member. Preferably, the opening (1g) is present on one side of the housing wall (26) whereas the gear wheel (1a) and protrusion (1b) are on located on the other side of the wall (26) together with the needle holder (3), NCE (2) and spike carrier (6). Figure 5c depicts a view from the top of the assembly.

The functioning of the needle insertion and retraction mechanism is explained using Figures 6 to 11. The assembly of the cam shaft (1) and needle control element (2) is shown in Figure 6a, the protruding part of the cam shaft (1), comprising the gear wheel (1a) and protrusion (1b), is inserted into the cavity formed in the front surface (2a) of the NCE. The gear teeth (1f) of the cam shaft (1) are not engaged with the gear teeth (2e) of the gear rack (2b) of the needle control element. In Figures 6b and 6c, the cross sectional views through planes A-A (first layer) and B-B (second layer) are shown respectively. In the initial position of the NCE, prior to activation of the device, the protrusion (1b) of the cam shaft (1) engages the curved groove (2c) of the NCE (Figure 6b), while the gear wheel (1a) is not engaged with the gear rack (2b), Figure 6c. The needle holder (3) with counter arrester (3a) as presented in Figure 6b abuts the arrester (2i) of the NCE. A rotation around the first axis (45) in the direction indicated by the arrow would release the abutment between the needle holder and the NCE and start in needle insertion. This needle insertion is prevented due to the link-motion (1b, 2c), the protrusion (1b) of the cam shaft abuts the side wall of the curved groove (2c) and this abutment is indicated in Figure 6b (47). Thus during storage the needle control element (2) is prevented from rotation thereby securing the needle holder (3) in the needle retracted position.

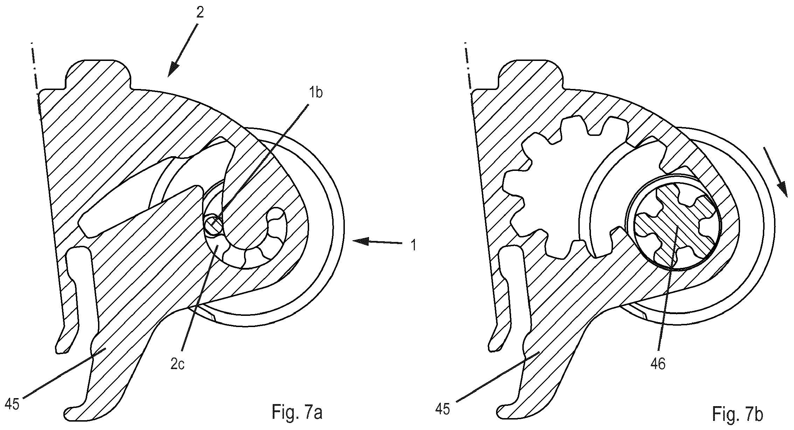

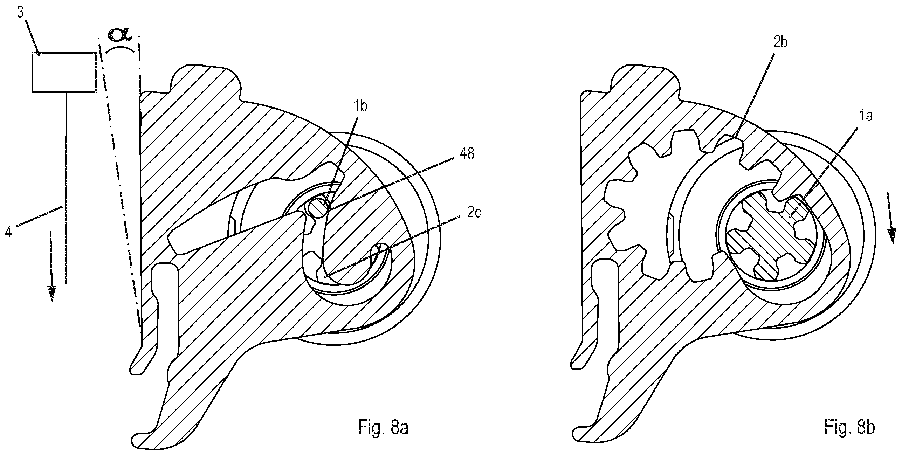

After activation of the device, the patient pushes the button (42) and the cam shaft (1) is rotated by the electromotor in the first rotation direction around the second axis (46) as indicated by the arrow in Figure 7b. The protrusion (1b) of the cam shaft (1) runs through the curved groove (2c) of the NCE and the NCE remains in the initial position of Figures 6a,6b,6c, and therewith also the needle holder (3) remains in the needle retracted position (Figure 7a). Once the curved section of the groove (2c) ends, the protrusion (1b) will abut (48) the side wall of the groove upon continued rotation of the cam shaft (1) and consequently the needle control element (2) will start rotating around the first axis (45), Figure 8a. The initial position of the needle control element is indicated with a dotted line. Rotation of the cam shaft (1) over a first angle ensures that the needle control element (2) is rotated over a second angle (α, Figure 8a). In this position of the NCE, the abutment between the needle holder (3) and the arrester (2i) is released and the needle holder (3) with the needle (4) will move towards the needle inserted position due to the bias of the spring (5) and/or the link-motion (6a,3d), see Figures 5a and 5c.

In the example shown in Figure 5, the release of the abutment between the NCE and the needle holder (3) ensures that the spike carrier (6) can move parallel to the bottom surface of the device, penetrate the septum of the cartridge (20) and establish a fluid connection to the liquid medicament. Simultaneously, the link- motion (6a, 3d) drives the needle holder (3) towards the needle inserted position. In this example the needle holder (3) is indirectly biased by the spring (5) via the link-motion (6a, 3d) that exists between the spike carrier (6) and the needle holder (3). Alternatively, the spring (5) may act directly onto the needle holder (3). During the needle insertion, the gear wheel (1a) of the cam shaft (1) is not engaged with the gear rack (2b), Figure 8b. After the needle insertion, the cartridge is emptied before the needle (4) is retracted from the skin back into the device. Medication delivery, either as a bolus, and/or as a basal rate is controlled by the delivery mechanism with the control unit (30). Preferably, the piston rod (37) is advanced which in turn advances the plug or stopper in the cartridge. The drive mechanism may be driven by the electromotor rotating in a rotation direction that is opposite to the first rotating direction that is used for rotating the cam shaft (1) and inserting the needle. This requires a reversal of the rotation direction of the electromotor after the needle insertion. Once the cartridge (20) has been emptied, the needle (4) needs to be retracted into the device and this may require an additional reversal of the rotation direction, e.g. equal to the first rotation direction used for insertion of the needle.

Using the link-motion (1b, 2c) for rotating the NCE has the advantage that the relative rotational play between the protrusion (1c) and the curved groove (2c) can be high before the NCE is rotated, compare Figures 6 and 7.

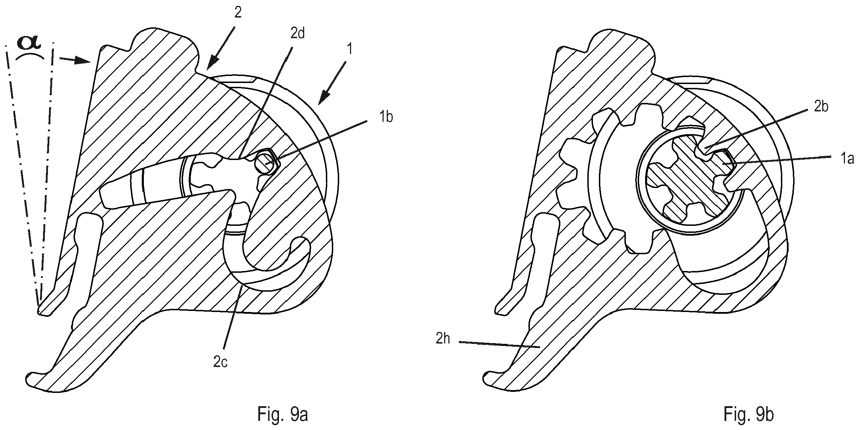

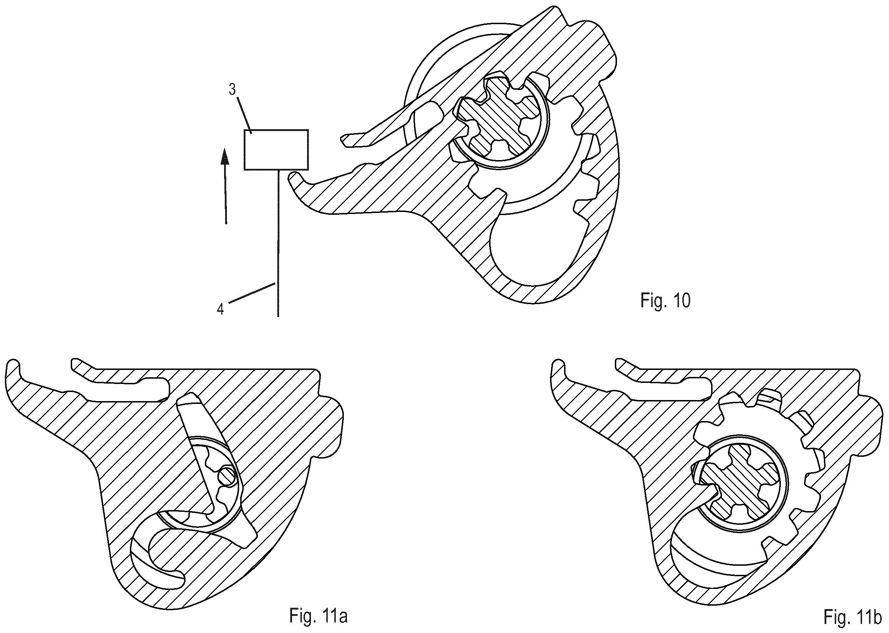

The retraction of the needle is shown in Figures 9 to 11. When the cam shaft (1) is rotated further (through a third) angle, then the gear wheel (1a) of the cam shaft engages the gear rack (2b) of the NCE, see Figure 9b. Thus until now, the rotation of the needle control element by the cam shaft was dictated by the link-motion (1b, 2c) which is positioned at cross section A-A in the first layer (21), e.g. furthest away from the front surface (2a) of the NCE. Once the gear wheel (1a) and gear rack (2b) get into engagement, the gearing engagement (1a, 2b) is activated which is positioned in a plane closer to the front surface (2a), or the second layer (2m) of the NCE as indicated by cross section level B-B (Figure 9b). The gearing engagement (2b, 1a) has the advantage of an efficient and direct transfer of the rotational torque of the cam shaft (1) to the NCE without rotational play. Further rotation of the cam-shaft (1) in the first rotation direction rotates the NCE (Figure 9) and the needle retraction arm (2h) will abut the needle holder such that the needle holder (3) is moved from the inserted towards the retracted position. Simultaneously, the link-motion engagement (1b, 2c) is released as the protrusion (1b) of the cam shaft enters the release section (2d) of the needle control element (Figure 9a). Therewith the link motion (1b, 2c) cannot interfere with the gearing engagement (2b, 1a). Finally, the needle control element (2) is in the needle retracted position as shown in Figure 11, and the device can be removed from the skin without the danger of undesired needle stitching by the patient.

**Part Annotation**

| | |
|---|---|
| | (6c) third guide slot |
| (1) cam shaft | (7) spike |
| (1a) gear wheel | (8) tubing |
| (1b) protrusion | (9) peel foil |
| (1c) circumferential groove | (20) cartridge, reservoir |
| (1d) cylindrical body | (21) crimp/septum |
| (1 e) end wall | (22) shoulder |
| (1f) gear teeth | (23) cartridge holder |
| (1g) opening | (24) housing fluid path unit |
| (2) needle control element (NCE) | (25) cartridge fixator |
| (2a) front surface | (26) wall housing |
| (2b) gear rack | (27) bottom section housing |
| (2c) curved grooved | (28) housing cover |
| (2d) release section | (30) control unit |
| (2e) gear teeth | (31) battery |
| (2f) protrusion | (32) motor |
| (2g) locking arm | (33) threaded rod |
| (2h) retraction arm | (34) gear wheel |
| (2i) arrester | (35) shaft |
| (2j) protrusion | (36) coupling member |
| (2k) back surface | (37) segmented piston rod |
| (21) first layer | (38) carrier part |
| (2m) second layer | (39) cover carrier part |
| (3) needle holder | (40) guidance piston rod |
| (3a) counter arrester | (41) housing cover |
| (3b) second guidance | (42) button |
| (3c) second guidance | (43) fluid path unit |
| (3d) protrusion | (44) needle insertion mechanism |
| (4) needle | (45) first axis |
| 5) Biasing member, spring | (46) second axis |
| (6) spike carrier | (47) abutment |
| (6a) first guide slot | (48) abutment |
| (6b) second guide slot | (α) second angle |

## Claims

1. An injection or infusion device comprising a housing and a needle insertion and retraction mechanism, the needle insertion and retraction mechanism comprising:
a needle holder for holding a needle or cannula,
a needle control element being rotatable with respect to the housing around a first axis and having a groove formed in a first layer of the needle control element that is perpendicular to the first axis,
a cam-shaft being rotatable around a second axis with respect to the housing that is parallel to the first axis,
the cam shaft comprises a protrusion engaging a first section of the groove of the needle control element to form a link-motion such that the needle control element is prevented from rotation around the first axis, thereby keeping the needle holder in abutment with an arrester that is part of the needle control element and thereby keeping the needle holder in a needle retracted position against the bias of a spring force,
**characterized in that**
the needle control element has a curved gear rack formed in a second layer of the needle control element that is perpendicular to the first axis of the needle control element,
and the cam-shaft comprises a gear wheel engageable to the gear rack of the needle control element.

2. The injection or infusion device comprising a housing and a needle insertion and retraction mechanism according to claim 1 whereby the needle control element is preferably disc shaped and wherein the first layer and second layer are in a series arrangement and the cam shaft is positioned adjacent to the second layer.

3. The injection or infusion device comprising a housing and a needle insertion and retraction mechanism according to any of the previous claims whereby the gear wheel is disengaged from the curved gear rack when the needle holder is in abutment with the arrester of the needle control element.

4. The injection or infusion device comprising a housing and a needle insertion and retraction mechanism according to the previous claims wherein the first section of the groove of the needle control element is curved or partially circular shaped.

5. The needle insertion and retraction mechanism according to any of the previous claims wherein the needle control element is prevented from rotation around the first axis by abutment of the protrusion of the cam shaft with a side wall of the first section of the groove of the needle control element.

6. The needle insertion and retraction mechanism according to any of the previous claims wherein a rotation of the cam shaft in a first direction through a first angle rotates the needle control element through a second angle due to the link-motion between the needle control element and the cam shaft.

7. The needle insertion and retraction mechanism according to claim 6, wherein the rotation of the needle control element through the second angle releases the abutment between the arrester of the needle control element and the needle holder whereby the needle holder is moved from the needle retracted to a needle inserted position by the spring force.

8. The needle insertion and retraction mechanism according to claims 6 or 7 wherein rotation of the cam shaft in the first direction through a third angle which is beyond the first angle brings the gear wheel of the cam shaft into a gearing engagement with the curved gear rack of the needle control element due to the link-motion.

9. The needle insertion and retraction mechanism according to claim 8 whereby the link-motion between the protrusion of the cam shaft and the first section of the groove of the needle control element is released and the protrusion enters a second section of the groove.

10. The needle insertion and retraction mechanism according to claim 8 wherein the gearing engagement rotates the needle control element beyond the second angle such that a retraction arm preferably being part of the needle control element moves the needle holder from the needle inserted position back to the needle retracted position.

11. The needle insertion and retraction mechanism according to any of the previous claims wherein the needle control element is engaged with the housing or a housing part of the injection or infusion device.

12. The needle insertion and retraction mechanism according to claim 11 wherein the needle control element has at least one protrusion oriented parallel to the first axis and preferably defining the first axis, the protrusion engaging a recess in the housing or a part of the housing to form a bearing such that the needle control element can rotate around the first axis.

13. The needle insertion and retraction mechanism according to any of the previous claims wherein the cam-shaft has a cylindrical shape having an opening on one side and the protrusion and gear wheel being positioned opposite to the opening along the second axis, the protrusion of the cam shaft engaging the groove of the needle control element is oriented parallel to the second axis and positioned preferably off-axis to the second axis.

14. The needle insertion and retraction mechanism according to claims 8 to 13 wherein the cam shaft is rotated in a second rotation direction which is opposite to the first rotation direction, the cam shaft being rotated in the second rotation direction between the first angle of rotation and third angle of rotation of the cam shaft.
